# EUROPEAN PATENT APPLICATION

(11) **EP 3 323 426 A1**
(43) Date of publication of application: **23.05.2018**
(21) Application number: 16199413.2
(22) Date of filing: 17.11.2016
(51) Int. Cl.: A61K 39/02

(54) **IMMUNOGENIC AND VACCINE COMPOSITIONS FOR USE AGAINST BORDETELLA BRONCHISEPTICA INFECTION**

(71) Applicant: Ecole Nationale Vétérinaire de Toulouse, 31300 Toulouse (FR); Cyanimal IP, 31280 Dremil-Lafage (FR)
(72) Inventor: NALIN, Renaud, 31280 Drémil Lafage (FR); BOULLIER, Séverine, 31100 Toulouse (FR); BOUSQUET-MELOU, Alain, 31500 Toulouse (FR); CORNILLE, Patrick, 13760 Saint-Cannat (FR)
(74) Representative: Cabinet Plasseraud

(57) **Abstract**

The present invention generally relates to immunogenic composition comprising *Bordetella sp.* Adenylate cyclase toxin (CyaA) for use for treating *Bordetella bronchiseptica* infections in non-human animals.

More particularly, the invention relates to immunogenic composition comprising *Bordetella sp.* Adenylate cyclase toxin (CyaA) vector for use for treating *Bordetella bronchiseptica* infections in non-human animals.

The present invention also relate to an immunogenic composition comprising a *Bordetella sp.* adenylate cyclase toxin (CyaA) vector carrying at least one antigen of *Bordetella bronchiseptica.*

SEQUENCE LISTING

<110>  E.N.V.T. et al

<120>  Immunogenic and vaccine compositions for use against Bordetella
       bronchiseptica infection

<130>  MA/MGU - BET160312 US

<160>  6

<170>  PatentIn version 3.5

<210>  1
<211>  1706
<212>  PRT
<213>  Bordetella pertussis

<400>  1

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala

675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                 1005

His Val  Gln His Ile Ile Gly  Gly Ala Gly Asn Asp  Ser Ile Thr
    1010                 1015                 1020

Gly Asn  Ala His Asp Asn Phe  Leu Ala Gly Gly Ser  Gly Asp Asp
    1025                 1030                 1035

Arg Leu  Asp Gly Gly Ala Gly  Asn Asp Thr Leu Val  Gly Gly Glu
    1040                 1045                 1050

Gly Gln  Asn Thr Val Ile Gly  Gly Ala Gly Asp Asp  Val Phe Leu
    1055                 1060                 1065

Gln Asp  Leu Gly Val Trp Ser  Asn Gln Leu Asp Gly  Gly Ala Gly
    1070                 1075                 1080

Val Asp  Thr Val Lys Tyr Asn  Val His Gln Pro Ser  Glu Glu Arg
    1085                 1090                 1095

Leu Glu  Arg Met Gly Asp Thr  Gly Ile His Ala Asp  Leu Gln Lys
    1100                 1105                 1110

Gly Thr  Val Glu Lys Trp Pro  Ala Leu Asn Leu Phe  Ser Val Asp
    1115                 1120                 1125

His Val  Lys Asn Ile Glu Asn  Leu His Gly Ser Arg  Leu Asn Asp
    1130                 1135                 1140

Arg Ile  Ala Gly Asp Asp Gln  Asp Asn Glu Leu Trp  Gly His Asp
    1145                 1150                 1155

Gly Asn  Asp Thr Ile Arg Gly  Arg Gly Gly Asp Asp  Ile Leu Arg
    1160                 1165                 1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Val Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val

1640                1645                1650

Glu Ile  Ile His Ala Ala Asn  Gln Ala Val Asp Gln  Ala Gly Ile
    1655                1660                1665

Glu Lys  Leu Val Glu Ala Met  Ala Gln Tyr Pro Asp  Pro Gly Ala
    1670                1675                1680

Ala Ala  Ala Ala Pro Pro Ala  Ala Arg Val Pro Asp  Thr Leu Met
    1685                1690                1695

Gln Ser  Leu Ala Val Asn Trp  Arg
    1700                1705

<210>  2
<211>  1706
<212>  PRT
<213>  Bordetella bronchiseptica

<400>  2

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asn Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala

145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Pro Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Gly Gly Arg Ser Lys Ser Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Ala
785                 790                 795                 800

Asp Val Phe Val Asp Arg Phe Ile Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Glu Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Lys Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                1005

His Val  Gln His Ile Ile Gly  Gly Ala Gly Asn Asp  Ser Ile Thr
    1010                 1015                 1020

Gly Asn  Ala His Asp Asn Phe  Leu Ala Gly Gly Ala  Gly Asp Asp
    1025                 1030                 1035

Arg Leu  Asp Gly Gly Ala Gly  Asn Asp Thr Leu Val  Gly Gly Glu
    1040                 1045                 1050

Gly His  Asn Thr Val Val Gly  Gly Ala Gly Asp Asp  Val Phe Leu
    1055                 1060                 1065

Gln Asp  Leu Gly Val Trp Ser  Asn Gln Leu Asp Gly  Gly Ala Gly
    1070                 1075                 1080

Val Asp  Thr Val Lys Tyr Asn  Val His Gln Pro Ser  Glu Glu Arg
    1085                 1090                 1095

Leu Glu  Arg Met Gly Glu Thr  Gly Ile His Ala Asp  Leu Gln Lys
    1100                 1105                 1110

Gly Thr  Val Glu Lys Trp Pro  Ala Leu Asn Leu Phe  Ser Val Asp
    1115                 1120                 1125

His Val  Lys Asn Ile Glu Asn  Leu His Gly Ser Ser  Leu Asn Asp
    1130                 1135                 1140

Ser Ile  Ala Gly Asp Asp Arg  Asp Asn Glu Leu Trp  Gly Asp Asp

1145                1150                1155

Gly Asn Asp Thr Ile His Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Ala
    1205                1210                1215

Gly Lys Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Ser Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Pro Ala Arg
    1325                1330                1335

Glu His Asp Val Leu Arg Gly Gly Ala Gly Val Asp Thr Val Asp
    1340                1345                1350

Tyr Ser Gln Ala Gly Ala His Ala Gly Val Ala Thr Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Glu Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Asn Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg His Ile Glu Asn Ala Val Gly Ser Val Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Ala Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ala Ile His Ala Ala Asn Gln Ala Ile Asp Pro Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

<210> 3
<211> 1706
<212> PRT
<213> Bordetella parapertussis

<400> 3

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Gly Gly Arg Ser Lys Ser Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Val Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala

625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Ala
785                 790                 795                 800

Asp Val Phe Val Asp Arg Phe Ile Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Glu Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Lys Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                1005

His Val  Gln His Ile Ile Gly  Gly Ala Gly Asn Asp  Ser Ile Thr
    1010                1015                1020

Gly Asn  Ala His Asp Asn Phe  Leu Ala Gly Gly Ala  Gly Asp Asp
    1025                1030                1035

Arg Leu  Asp Gly Gly Ala Gly  Asn Asp Thr Leu Val  Gly Gly Glu
    1040                1045                1050

Gly His  Asn Thr Val Val Gly  Gly Ala Gly Asp Asp  Val Phe Leu
    1055                1060                1065

Gln Asp  Leu Gly Val Trp Ser  Asn Gln Leu Asp Gly  Gly Ala Gly
    1070                1075                1080

Val Asp  Thr Val Lys Tyr Asn  Val His Gln Pro Ser  Glu Glu Arg
    1085                1090                1095

Leu Glu  Arg Met Gly Asp Thr  Gly Ile His Ala Asp  Leu Gln Lys
    1100                1105                1110

Gly Thr  Val Glu Lys Trp Pro  Ala Leu Asn Leu Phe  Ser Val Asp
    1115                1120                1125

His Val Lys Asn Ile Glu Asn Leu His Gly Ser Ser Leu Asn Asp
    1130                1135                1140

Ser Ile Ala Gly Asp Asp Arg Asp Asn Glu Leu Trp Gly Asp Asp
    1145                1150                1155

Gly Asn Asp Thr Ile His Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Ala
    1205                1210                1215

Gly Lys Ile Val Ala Pro His Glu Tyr Gly Phe Gly Ile Glu Ala
    1220                1225                1230

Asp Leu Ser Glu Gly Trp Val Arg Lys Ala Ala Arg Arg Gly Met
    1235                1240                1245

Gly Tyr Tyr Asp Ser Val Arg Ser Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Pro Ala Arg
    1325                1330                1335

Glu His Asp Val Leu Arg Gly Gly Ala Gly Val Asp Thr Val Asp
    1340                1345                1350

Tyr Ser Gln Ala Gly Ala His Ala Gly Val Ala Thr Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Glu Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Asn Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg His Ile Glu Asn Ala Val Gly Ser Val Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Ala Gly Tyr Gly His Asp Thr Ile Tyr Glu Ser Gly Gly Gly His

1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ala Ile His Ala Ala Asn Gln Ala Ile Asp Pro Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

<210>  4
<211>  232
<212>  PRT
<213>  Bordetella bronchiseptica

<400>  4

Met Val Pro His His His His His His Ser Arg Ala Trp Arg His Pro
1               5                   10                  15

Gln Phe Gly Gly His His His His His His Ala Ser Thr Ile Asp Leu
            20                  25                  30

Gly Val Ser Leu Thr Ser Gln Ala Gly Gly Leu Gln Gly Ile Asp Leu
        35                  40                  45

Lys Ser Met Asp Ile Gln Thr Leu Met Val Tyr Val Gln Gly Arg Arg
    50                  55                  60

Ala Glu Leu Leu Thr Ala Gln Met Gln Thr Gln Ala Glu Val Val Gln
65                  70                  75                  80

Lys Ala Asn Glu Arg Met Ala Gln Leu Asn Glu Val Leu Ser Ala Leu
                85                  90                  95

Ser Arg Ala Lys Ala Glu Phe Pro Pro Asn Pro Lys Pro Gly Asp Thr

            100                 105                 110

Ile Pro Gly Trp Asp Asn Gln Lys Val Ser Arg Ile Glu Val Pro Leu
        115                 120                 125

Asn Asp Ala Leu Arg Ala Ala Gly Leu Thr Gly Met Phe Glu Ala Arg
    130                 135                 140

Asp Gly Gln Val Thr Ala Pro Gly Gly Arg Gly Thr Gln Val Val Asn
145                 150                 155                 160

Gly Thr Gly Val Met Ala Gly Ser Thr Thr Tyr Lys Glu Leu Glu Ser
                165                 170                 175

Ala Tyr Thr Thr Val Lys Gly Met Leu Asp Thr Ala Ser Asn Thr Gln
            180                 185                 190

Gln Met Asp Met Ile Arg Leu Gln Ala Ala Ser Asn Lys Arg Asn Glu
        195                 200                 205

Ala Phe Glu Val Met Thr Asn Thr Glu Lys Arg Arg Ser Asp Leu Asn
    210                 215                 220

Ser Ser Ile Thr Asn Asn Met Arg
225                 230

<210> 5
<211> 249
<212> PRT
<213> Bordetella bronchiseptica

<400> 5

Met Gly Thr Val Asn Gly Leu Glu Gln Leu Glu Ser Ile Ile Asn Phe
1               5                   10                  15

Glu Lys Leu Thr Glu Trp Thr Ser Ser Asn Val Met Glu Glu Arg Lys
            20                  25                  30

Ile Lys Val Tyr Leu Pro Arg Ala Arg Ala Ala Thr Ser Thr Ile Asp
        35                  40                  45

Leu Gly Val Ser Leu Thr Ser Gln Ala Gly Gly Leu Gln Gly Ile Asp
    50                  55                  60

Leu Lys Ser Met Asp Ile Gln Thr Leu Met Val Tyr Val Gln Gly Arg
65                  70                  75                  80

Arg Ala Glu Leu Leu Thr Ala Gln Met Gln Thr Gln Ala Glu Val Val

85                  90                  95

Gln Lys Ala Asn Glu Arg Met Ala Gln Leu Asn Glu Val Leu Ser Ala
            100                 105                 110

Leu Ser Arg Ala Lys Ala Glu Phe Pro Pro Asn Pro Lys Pro Gly Asp
        115                 120                 125

Thr Ile Pro Gly Trp Asp Asn Gln Lys Val Ser Arg Ile Glu Val Pro
    130                 135                 140

Leu Asn Asp Ala Leu Arg Ala Ala Gly Leu Thr Gly Met Phe Glu Ala
145                 150                 155                 160

Arg Asp Gly Gln Val Thr Ala Pro Gly Gly Arg Gly Thr Gln Val Val
                165                 170                 175

Asn Gly Thr Gly Val Met Ala Gly Ser Thr Thr Tyr Lys Glu Leu Glu
            180                 185                 190

Ser Ala Tyr Thr Thr Val Lys Gly Met Leu Asp Thr Ala Ser Asn Thr
        195                 200                 205

Gln Gln Met Asp Met Ile Arg Leu Gln Ala Ala Ser Asn Lys Arg Asn
    210                 215                 220

Glu Ala Phe Glu Val Met Thr Asn Thr Glu Lys Arg Arg Ser Asp Leu
225                 230                 235                 240

Asn Ser Ser Ile Thr Asn Asn Met Arg
                245

<210>  6
<211>  1590
<212>  PRT
<213>  Bordetella bronchiseptica

<400>  6

Met Gly Thr Val Asn Gly Leu Glu Gln Leu Glu Ser Ile Ile Asn Phe
1               5                   10                  15

Glu Lys Leu Thr Glu Trp Thr Ser Ser Asn Val Met Glu Glu Arg Lys
            20                  25                  30

Ile Lys Val Tyr Leu Pro Arg Ala Arg Ala Ala Thr Ser Thr Ile Asp
        35                  40                  45

Leu Gly Val Ser Leu Thr Ser Gln Ala Gly Gly Leu Gln Gly Ile Asp

50                  55                  60

Leu Lys Ser Met Asp Ile Gln Thr Leu Met Val Tyr Val Gln Gly Arg
65                  70                  75                  80

Arg Ala Glu Leu Leu Thr Ala Gln Met Gln Thr Gln Ala Glu Val Val
                85                  90                  95

Gln Lys Ala Asn Glu Arg Met Ala Gln Leu Asn Glu Val Leu Ser Ala
            100                 105                 110

Leu Ser Arg Ala Lys Ala Glu Phe Pro Pro Asn Pro Lys Pro Gly Asp
        115                 120                 125

Thr Ile Pro Gly Trp Asp Asn Gln Lys Val Ser Arg Ile Glu Val Pro
    130                 135                 140

Leu Asn Asp Ala Leu Arg Ala Ala Gly Leu Thr Gly Met Phe Glu Ala
145                 150                 155                 160

Arg Asp Gly Gln Val Thr Ala Pro Gly Gly Arg Gly Thr Gln Val Val
                165                 170                 175

Asn Gly Thr Gly Val Met Ala Gly Ser Thr Thr Tyr Lys Glu Leu Glu
            180                 185                 190

Ser Ala Tyr Thr Thr Val Lys Gly Met Leu Asp Thr Ala Ser Asn Thr
        195                 200                 205

Gln Gln Met Asp Met Ile Arg Leu Gln Ala Ala Ser Asn Lys Arg Asn
    210                 215                 220

Glu Ala Phe Glu Val Met Thr Asn Thr Glu Lys Arg Arg Ser Asp Leu
225                 230                 235                 240

Asn Ser Ser Ile Thr Asn Asn Met Arg Thr Gly Ala Ala Pro Ser Arg
                245                 250                 255

Ser Lys Phe Ser Pro Asp Val Leu Glu Thr Val Pro Ala Ser Pro Gly
            260                 265                 270

Leu Arg Arg Pro Ser Leu Gly Ala Val Glu Arg Gln Asp Ser Gly Tyr
        275                 280                 285

Asp Ser Leu Asp Gly Val Gly Ser Arg Ser Phe Ser Leu Gly Glu Val
    290                 295                 300

Ser Asp Met Ala Ala Val Glu Ala Ala Glu Leu Glu Met Thr Arg Gln
305                 310                 315                 320

Val Leu His Ala Gly Ala Arg Gln Asp Asp Ala Glu Pro Gly Val Ser
                325                 330                 335

Gly Ala Ser Ala His Trp Gly Gln Arg Ala Leu Gln Gly Ala Gln Ala
            340                 345                 350

Val Ala Ala Ala Gln Arg Leu Val His Ala Ile Ala Leu Met Thr Gln
        355                 360                 365

Phe Gly Arg Ala Gly Ser Thr Asn Thr Pro Gln Glu Ala Ala Ser Leu
    370                 375                 380

Ser Ala Ala Val Phe Gly Leu Gly Glu Ala Ser Ser Ala Val Ala Glu
385                 390                 395                 400

Thr Val Ser Gly Phe Phe Arg Gly Ser Ser Arg Trp Ala Gly Gly Phe
                405                 410                 415

Gly Val Ala Gly Gly Ala Met Ala Leu Gly Gly Gly Ile Ala Ala Ala
            420                 425                 430

Val Gly Ala Gly Met Ser Leu Thr Asp Asp Ala Pro Ala Gly Gln Lys
        435                 440                 445

Ala Ala Ala Gly Ala Glu Ile Ala Leu Gln Leu Thr Gly Gly Thr Val
    450                 455                 460

Glu Leu Ala Ser Ser Ile Ala Leu Ala Leu Ala Ala Ala Arg Gly Val
465                 470                 475                 480

Thr Ser Gly Leu Gln Val Ala Gly Ala Ser Ala Gly Ala Ala Ala Gly
                485                 490                 495

Ala Leu Ala Ala Ala Leu Ser Pro Met Glu Ile Tyr Gly Leu Val Gln
            500                 505                 510

Gln Ser His Tyr Ala Asp Gln Leu Asp Lys Leu Ala Gln Glu Ser Ser
        515                 520                 525

Ala Tyr Gly Tyr Glu Gly Asp Ala Leu Leu Ala Gln Leu Tyr Arg Asp
    530                 535                 540

Lys Thr Ala Ala Glu Gly Ala Val Ala Gly Val Ser Ala Val Leu Ser
545                 550                 555                 560

Thr Val Gly Ala Ala Val Ser Ile Ala Ala Ala Ala Ser Val Val Gly
                565                 570                 575

Ala Pro Val Ala Val Val Thr Ser Leu Leu Thr Gly Ala Leu Asn Gly
            580                 585                 590

Ile Leu Arg Gly Val Gln Gln Pro Ile Ile Glu Lys Leu Ala Asn Asp
        595                 600                 605

Tyr Ala Arg Lys Ile Asp Glu Leu Gly Gly Pro Gln Ala Tyr Phe Glu
    610                 615                 620

Lys Asn Leu Gln Ala Arg His Glu Gln Leu Ala Asn Ser Asp Gly Leu
625                 630                 635                 640

Arg Lys Met Leu Ala Asp Leu Gln Ala Gly Trp Asn Ala Ser Ser Val
                645                 650                 655

Ile Gly Val Gln Thr Thr Glu Ile Ser Lys Ser Ala Leu Glu Leu Ala
            660                 665                 670

Ala Ile Thr Gly Asn Ala Asp Asn Leu Lys Ser Val Asp Val Phe Val
        675                 680                 685

Asp Arg Phe Val Gln Gly Glu Arg Val Ala Gly Gln Pro Val Val Leu
    690                 695                 700

Asp Val Ala Ala Gly Gly Ile Asp Ile Ala Ser Arg Lys Gly Glu Arg
705                 710                 715                 720

Pro Ala Leu Thr Phe Ile Thr Pro Leu Ala Ala Pro Gly Glu Glu Gln
                725                 730                 735

Arg Arg Arg Thr Lys Thr Gly Lys Ser Glu Phe Thr Thr Phe Val Glu
            740                 745                 750

Ile Val Gly Lys Gln Asp Arg Trp Arg Ile Arg Asp Gly Ala Ala Asp
        755                 760                 765

Thr Thr Ile Asp Leu Ala Lys Val Val Ser Gln Leu Val Asp Ala Asn
    770                 775                 780

Gly Val Leu Lys His Ser Ile Lys Leu Asp Val Ile Gly Gly Asp Gly
785                 790                 795                 800

Asp Asp Val Val Leu Ala Asn Ala Ser Arg Ile His Tyr Asp Gly Gly
                805                 810                 815

Ala Gly Thr Asn Thr Val Ser Tyr Ala Ala Leu Gly Arg Gln Asp Ser
            820                 825                 830

Ile Thr Val Ser Ala Asp Gly Glu Arg Phe Asn Val Arg Lys Gln Leu
        835                 840                 845

Asn Asn Ala Asn Val Tyr Arg Glu Gly Val Ala Thr Gln Thr Thr Ala
    850                 855                 860

Tyr Gly Lys Arg Thr Glu Asn Val Gln Tyr Arg His Val Glu Leu Ala
865                 870                 875                 880

Arg Val Gly Gln Val Val Glu Val Asp Thr Leu Glu His Val Gln His
                885                 890                 895

Ile Ile Gly Gly Ala Gly Asn Asp Ser Ile Thr Gly Asn Ala His Asp
            900                 905                 910

Asn Phe Leu Ala Gly Gly Ser Gly Asp Asp Arg Leu Asp Gly Gly Ala
        915                 920                 925

Gly Asn Asp Thr Leu Val Gly Gly Glu Gly Gln Asn Thr Val Ile Gly
    930                 935                 940

Gly Ala Gly Asp Asp Val Phe Leu Gln Asp Leu Gly Val Trp Ser Asn
945                 950                 955                 960

Gln Leu Asp Gly Gly Ala Gly Val Asp Thr Val Lys Tyr Asn Val His
                965                 970                 975

Gln Pro Ser Glu Glu Arg Leu Glu Arg Met Gly Asp Thr Gly Ile His
            980                 985                 990

Ala Asp Leu Gln Lys Gly Thr Val Glu Lys Trp Pro Ala Leu Asn Leu
        995                 1000                1005

Phe Ser Val Asp His Val Lys Asn Ile Glu Asn Leu His Gly Ser
    1010                1015                1020

Arg Leu Asn Asp Arg Ile Ala Gly Asp Asp Gln Asp Asn Glu Leu
    1025                1030                1035

Trp Gly His Asp Gly Asn Asp Thr Ile Arg Gly Arg Gly Gly Asp
    1040                1045                1050

Asp Ile Leu Arg Gly Gly Leu Gly Leu Asp Thr Leu Tyr Gly Glu

1055                1060                1065

Asp Gly Asn Asp Ile Phe Leu Gln Asp Asp Glu Thr Val Ser Asp
    1070                1075                1080

Asp Ile Asp Gly Gly Ala Gly Leu Asp Thr Val Asp Tyr Ser Ala
    1085                1090                1095

Met Ile His Pro Gly Arg Ile Val Ala Pro His Glu Tyr Gly Phe
    1100                1105                1110

Gly Ile Glu Ala Asp Leu Ser Arg Glu Trp Val Arg Lys Ala Ser
    1115                1120                1125

Ala Leu Gly Val Asp Tyr Tyr Asp Asn Val Arg Asn Val Glu Asn
    1130                1135                1140

Val Ile Gly Thr Ser Met Lys Asp Val Leu Ile Gly Asp Ala Gln
    1145                1150                1155

Ala Asn Thr Leu Met Gly Gln Gly Gly Asp Asp Thr Val Arg Gly
    1160                1165                1170

Gly Asp Gly Asp Asp Leu Leu Phe Gly Gly Asp Gly Asn Asp Met
    1175                1180                1185

Leu Tyr Gly Asp Ala Gly Asn Asp Thr Leu Tyr Gly Gly Leu Gly
    1190                1195                1200

Asp Asp Thr Leu Glu Gly Gly Ala Gly Asn Asp Trp Phe Gly Gln
    1205                1210                1215

Thr Gln Ala Arg Glu His Asp Val Leu Arg Gly Gly Asp Gly Val
    1220                1225                1230

Asp Thr Val Asp Tyr Ser Gln Thr Gly Ala His Ala Gly Ile Ala
    1235                1240                1245

Ala Gly Arg Ile Gly Leu Gly Ile Leu Ala Asp Leu Gly Ala Gly
    1250                1255                1260

Arg Val Asp Lys Leu Gly Glu Ala Gly Ser Ser Ala Tyr Asp Thr
    1265                1270                1275

Val Ser Gly Ile Glu Asn Val Val Gly Thr Glu Leu Ala Asp Arg
    1280                1285                1290

Ile Thr Gly Asp Ala Gln Ala Asn Val Leu Arg Gly Ala Gly Gly
    1295                1300                1305

Ala Asp Val Leu Ala Gly Gly Glu Gly Asp Asp Val Leu Leu Gly
    1310                1315                1320

Gly Asp Gly Asp Asp Gln Leu Ser Gly Asp Ala Gly Arg Asp Arg
    1325                1330                1335

Leu Tyr Gly Glu Ala Gly Asp Asp Trp Phe Phe Gln Asp Ala Ala
    1340                1345                1350

Asn Ala Gly Asn Leu Leu Asp Gly Gly Asp Gly Arg Asp Thr Val
    1355                1360                1365

Asp Phe Ser Gly Pro Gly Arg Gly Leu Asp Ala Gly Ala Lys Gly
    1370                1375                1380

Val Phe Leu Ser Leu Gly Lys Gly Phe Ala Ser Leu Met Asp Glu
    1385                1390                1395

Pro Glu Thr Ser Asn Val Leu Arg Asn Ile Glu Asn Ala Val Gly
    1400                1405                1410

Ser Ala Arg Asp Asp Val Leu Ile Gly Asp Ala Gly Ala Asn Val
    1415                1420                1425

Leu Asn Gly Leu Ala Gly Asn Asp Val Leu Ser Gly Gly Ala Gly
    1430                1435                1440

Asp Asp Val Leu Leu Gly Asp Glu Gly Ser Asp Leu Leu Ser Gly
    1445                1450                1455

Asp Ala Gly Asn Asp Asp Leu Phe Gly Gly Gln Gly Asp Asp Thr
    1460                1465                1470

Tyr Leu Phe Gly Val Gly Tyr Gly His Asp Thr Ile Tyr Glu Ser
    1475                1480                1485

Gly Gly Gly His Asp Thr Ile Arg Ile Asn Ala Gly Ala Asp Gln
    1490                1495                1500

Leu Trp Phe Ala Arg Gln Gly Asn Asp Leu Glu Ile Arg Ile Leu
    1505                1510                1515

Gly Thr Asp Asp Ala Leu Thr Val His Asp Trp Tyr Arg Asp Ala
    1520                1525                1530

Asp His Arg Val Glu Ile Ile His Ala Ala Asn Gln Ala Val Asp
    1535                1540                1545

Gln Ala Gly Ile Glu Lys Leu Val Glu Ala Met Ala Gln Tyr Pro
    1550                1555                1560

Asp Pro Gly Ala Ala Ala Ala Ala Pro Pro Ala Ala Arg Val Pro
    1565                1570                1575

Asp Thr Leu Met Gln Ser Leu Ala Val Asn Trp Arg
    1580                1585                1590

## Description

### FIELD OF THE INVENTION

The present invention relates to the field of immunology, and in particular to the field of immunogenic and vaccine compositions. It relates to such compositions for use against *Bordetella bronchiseptica* infection and in particular against canine tracheobronchitis commonly named kennel cough.

### BACKGROUND OF THE INVENTION

*Bordetella bronchiseptica* (*B. bronchiseptica)* is a small, Gram-negative, rod-shaped bacterium of the genus Bordetella and closely related to *B. pertussis,* the obligate human pathogen that causes pertussis (whooping cough).

Humans are not natural carriers of *B. bronchiseptica,* which typically infects the respiratory tracts of animals and especially mammals animals such as cats, dogs, pigs, cattle, etc...

In veterinary medicine, *B. Bronchiseptica* alone but also combined with others pathogens leads to a range of pathologies in different non-human animal hosts such as:
In swine, *B. bronchiseptica* and *Pasteurella multocida* act synergistically to cause atrophic rhinitis, a disease resulting in arrested growth and distortion of the turbinate in the nasal terminus snout.

In cats, *B. bronchiseptica* causes tracheobronchitis, conjunctivitis, and rhinitis (upper respiratory tract infection - URI), mandibular lymphadenopathy, and pneumonia. However, URI can also be caused by herpesvirus, calicivirus, Mycoplasma or Chlamydia bacteria.

In dogs, *B. bronchiseptica* causes tracheobronchitis, commonly named kennel cough or bordetellosis, which is highly contagious respiratory disease typified by inflammation of the trachea and bronchi. The symptoms are a persistent cough, retching, watery nasal discharge and can be in severe cases, pneumonia, inappetence, fever, lethargy and even death. Even if *B. bronchiseptica* seems to be the major cause of this disease, others pathogens such as canine adenovirus, parainfluenza virus, and mycoplasma can also cause the symptoms alone or in combination.

*Bordetella* infection spreads through direct contact (licking, nuzzling), through the air (coughing or sneezing), or via contaminated fomites and can occur between animal species such as for example dogs and cats.

Vaccination of the non-human animals to prevent tracheobronchitis is commonly used but as and especially by vaccine against *B. bronchiseptica.*

The immunologic mechanism of protection provided by vaccination has not been well defined for *Bordetella bronchiseptica.* It is known that bacterial endotoxins play a role in causing pathology in both the upper respiratory tract as well as in the lung. Neutralization of those endotoxins by locally produced secretory antibody, most likely of the IgA class, as well as systemically produced IgG antibody that can reach the lung via the serum, would be important in reducing pathology caused by the toxins. It is also expected that secretions containing IgA and serum containing primarily IgG (but also some IgA) antibody to *Bordetella bronchiseptica* would provide protection in the lungs through transudation (Larson et al., Intern J Appl Res Vet Med, Vol. 11, No. 3, 2013).

There is currently three different types of *Bordetella bronchiseptica* vaccines licensed in the United States for use in dogs: a killed injectable vaccine for subcutaneous use, Bronchicine®CAe (Zoetis), a live attenuated vaccine for intranasal administration, Bronchi-shield®III (Boehringer Ingelheim Vetmedica), Vanguard® (Zoetis), Nobivac® (Merck Animal Health) and a live attenuated vaccine to be given orally, Bronchi-Shield®ORAL (Boehringer Ingelheim Vetmedica).

A comparative study has been made of protective immunity provided by oral, intranasal and parenteral Canine *Bordetella bronchiseptica* vaccines by Larson et al. The killed injectable vaccine Bronchicine®CAe was shown to significantly reduce clinical disease signs. The live attenuated intranasally administered vaccine, Bronchi-shield®III was considered superior in reducing clinical disease compared to the killed injectable vaccine. Live attenuated vaccines have the advantage to induce immunity after a single dose and have been shown to provide protection after only 72 hours. However, intranasal vaccines are more difficult to administer than an injectable product, and are therefore less popular with veterinarians. The oral *Bordetella* vaccine, Bronchi-Shield®ORAL, provided immunity that was similar to that induced by the intranasal product (Larson et al., Intern J Appl Res Vet Med, Vol. 11, No. 3, 2013).

Hence, in this challenge study, and also in previous vaccine studies where intranasally administered killed vaccines have been compared with systemically administered killed vaccines, protection from clinical disease has been greater with the live vaccines.

A disadvantage with live attenuated vaccines is that their protection is not really long lasting, so a second and possibly a third vaccination with a live attenuated vaccine must be necessary. Furthermore, while live attenuated vaccines induce local immunity, they may cause significant vaccination reaction, in particular in the respiratory tract after spray vaccination.

A general problem with intranasally given live attenuated vaccines is the fact that the vaccine does not stay in the nose, but leaks away through sneezing, leaking and the like. This is an unwanted situation for two reasons: most of the vaccine bacteria become spread into the environment, and an overdose must be given to assure a sufficient level of vaccination. These disadvantages are however accepted in practice since as motivated above intranasal vaccination is currently considered the only way to obtain at least partial protection.

Accordingly, there remains a need for an immunogenic composition, capable of being safely administered, which provides a long-acting immunoprotection against *Bordetella bronchiseptica* without deleterious side effects of interference.

### SUMMARY OF THE INVENTION

The present invention generally relates to immunogenic compositions comprising *Bordetella sp.* Adenylate cyclase toxin (CyaA) for use for treating *Bordetella bronchiseptica* infections in non-human animals.

More particularly, the invention relates to immunogenic compositions comprising *Bordetella sp.* Adenylate cyclase toxin (CyaA) vector for use for treating *Bordetella bronchiseptica* infections in non-human animals.

The *Bordetella sp.* adenylate cyclase toxin (CyaA) vector is capable of retaining the binding activity of native CyaA through its N-terminal domain into target cells and translocate into the cytosol of the antigen-presenting cells (APC). Hence, the adenylate cyclase toxin (CyaA) vector is suitable for delivering molecules, in particular *Bordetella bronchiseptica* antigens and for eliciting a protective immune response.

The present invention also relate to immunogenic compositions comprising a *Bordetella sp.* adenylate cyclase toxin (CyaA) vector carrying at least one antigen of *Bordetella bronchiseptica.*

### BRIEF DESCRIPTION OF THE DRAWINGS

**FIGURES1****:** Clinical follow-up of puppies after *Bordetella bronchiseptica* challenge.
**FIGURE 1A**: Clinical score determined every day for each dog after bacterial infection.
**FIGURE 1B****:** Comparison of the number of coughing/day between each group of puppies (control group and vaccine group)
**FIGURE 2****:** Specific IFNγ production by PBMC after CyaA stimulation determined by ELISpot in control (n=3) and vaccinated (n=4) group once a week after vaccination and infection. Results are presented as the mean of spot numbers (±SD) per 10⁶ cells for each group. Arrows indicate the times of vaccination and infection.
**FIGURE 3****:** Humoral response against CyaA
**FIGURE 3A****:** Total serum IgG specific for CyaA determined by ELISA. Results are presented as the mean (±SD) of OD obtained at serum dilutions of 1/20 000 for control (n=5) and vaccinated (n=10) dogs. P<0.01 for each time point between control and vaccinated dogs from D28 until the end of the experiment (D70).
**FIGURE 3B****:** IgG1 and IgG2 Abs specific for CyaA detected by ELISA in sera of vaccinated dogs (n=10) at D28, D49 and D70 after the first vaccine injection. Results are presented as the mean (±SD) of OD obtained at serum dilutions of 1/20 000.
**FIGURE 4****:** Immune response against Bsp22
**FIGURE 4A****:** Specific IFNγ production after Bsp22 stimulation by PBMC determined by ELISpot in control (n=3) and vaccinated dogs (n=4) once a week after vaccination and infection. Results are presented as the mean of spot numbers (±SD) per 10⁶ cells for each group. Arrows indicate the times of vaccination and infection.
**FIGURE 4B****:** Total serum IgG specific for Bsp 22 determined by ELISA. Results are presented as the mean (±SD) of OD obtained at serum dilutions of 1/20 000 for control (n=5) and vaccinated dogs (n=10).** P<0.01.
**FIGURE 4C****:** Serum IgG1 and IgG2 Abs specific for Bsp22 detected by ELISA in sera of vaccinated dogs (n=10) at D63 and D70 after the first vaccine injection. Results are presented as the mean (±SD) of OD obtained at serum dilutions of 1/20 000.

### DETAILLED DESCRIPTION

The present invention generally relates to an immunogenic composition comprising a *Bordetella sp.* adenylate cyclase toxin (CyaA) vector carrying at least one antigen of *Bordetella bronchiseptica.*

The term "immunogenic composition" or "vaccine" or its equivalents, relates to any composition comprising a *Bordetella sp.* adenylate cyclase toxin (CyaA) or a *Bordetella* sp. adenylate cyclase toxin (CyaA) vector, which can be used to elicit an immune response in a non-human animal. In one embodiment, the immunogenic composition is suitable for eliciting a T-cell immune response, and in a particular embodiment, Th1.
The immunogenic composition of the present invention can be used to treat a non-human animal susceptible to *Bordetella bronchiseptica* caused infections and/or diseases by means of administering the immunogenic composition. Advantageously, an immunogenic composition according to the invention induces a response orientated toward Th-1 phenotype, characterized by IFNγ production and IgG2 synthesis.

The Adenylate Cyclase toxin (CyaA) of Bordetella species in particular of *Bordetella pertussis* or *Bordetella bronchiseptica,* is a 1706 residue-long protein (177 kDa).

As used herein, " adenylate cyclase toxin" or "CyaA" or "native adenylate cyclase toxin (CyaA)" or "native CyaA" refer to the adenylate cyclase toxin protein from a *Bordetella sp.,* such as the adenylate cyclase toxin protein from *Bordetella pertussis* (Accession number CPI 05197, SEQ ID NO:1), the adenylate cyclase toxin protein from *Bordetella bronchiseptica* (Accession number KDS81064, SEQ ID NO:2), the adenylate cyclase toxin protein from *Bordetella hinzii* (Accession number AAY57201, SEQ ID NO:1), or the adenylate cyclase toxin protein *Bordetella parapertussis* (Accession number CAB76450, SEQ ID NO:3).

As used herein *Bordetella sp.* refers to *Bordetella species.*

In one preferred embodiment, the adenylate cyclase toxin (CyaA) is from *Bordetella pertussis* (SEQ ID NO: 1).

In another preferred embodiment, the adenylate cyclase toxin (CyaA) is from *Bordetella bronchiseptica* (SEQ ID NO: 2).

CyaA is a bifunctional protein that consists of an amino-terminal adenylate cyclase (AC) domain of about 400 residues (N-terminal part) and of a Repeats in ToXin (RTX) cytolysin moiety (Hly) of about 1300 residues (C-terminal part). The Hly moiety inserts into cellular membranes and mediates translocation of the enzymatic AC domain into cell cytosol, where this binds calmodulin and catalyzes conversion of ATP to cAMP, thus subverting cellular signaling. The AC domain appears to be a passive passenger that can be replaced by synthetic polypeptides that can also be delivered into cells by the Hly moiety.

The Adenylate cyclase toxin (CyaA) of *Bordetella* types, in particular of *Bordetella pertussis* has been described as a recombinant vector able to deliver efficiently polypeptides, such as antigens, into the cytosol of target sentinel cells commonly named Antigen Presenting Cells (APC).

The biology of the adenylate cyclase from *Bordetella pertussis,* studied at the Institut Pasteur, led to the discovery that this bacterial protein could be engineered into a potent antigen delivery vector as described in EP0637335 and EP17115047.

One of the main unique features of CyaA stems from its unique mechanism of penetrating into eukaryotic cells. CyaA is able to translocate its N-terminal domain directly across the plasma membrane of target cells, from the extracellular side into the cytosol (Guermonprez, P., Ladant, D., Karimova, G., Ullmann, A., and Leclerc, C. Direct delivery of the Bordetella pertussis adenylate cyclase toxin to the MHC class I antigen presentation pathway. J. Immunol. 1999;162, 1910-1916.; Bauche, C., Chenal, A., Knapp, O., Bodenreider, C., Benz, R., Chaffotte, A., and Ladant, D. Structural and functional characterization of an essential RTX subdomain of Bordetella pertussis adenylate cyclase toxin. J. Biol. Chem. 2006;281, 16914-16926 ; Vojtova-Vodolanova, J., Basler, M., Osicka, R., Knapp, O., Maier, E., Cerny, J., Benada, O., Benz, R., and Sebo, P. Oligomerization is involved in pore formation by Bordetella adenylate cyclase toxin. FASEB J. 2009;23, 2831-2843 ; Bumba, L., Masin, J., Fiser, R., and Sebo, P. Bordetella adenylate cyclase toxin mobilizes its 2 integrin receptor into lipid rafts to accomplish translocation across target cell membrane in two steps. PLoS Pathog. 2010;6, e1000901 ; Karst, J. C., Barker, R., Devi, U., Swann, M. J., Davi, M., Roser, S. J., Ladant, D., and Chenal, A. Identification of a region that assists membrane insertion and translocation of the catalytic domain of Bordetella pertussis CyaA toxin. J. Biol. Chem. 2012;287, 9200-9212 ; Subrini, O., Sotomayor-Pe'rez, A. C., Hessel, A., Spiaczka-Karst, J., Selwa, E., Sapay, N., Veneziano, R., Pansieri, J., Chopineau, J., Ladant, D., and Chenal, A. Characterization of a membrane-active peptide from the Bordetella pertussis CyaA toxin. J. Biol. Chem. 2013;288, 32585-32598 ; Uribe, K. B., Etxebarria, A., Mart 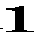 'n, C., and Ostolaza, H. Calpainmediated processing of adenylate cyclase toxin generates a cytosolic soluble catalytically active N-terminal domain. PLoS One. 2013;8, e67648 ; Veneziano, R., Rossi, C., Chenal, A., Devoisselle, J. M., Ladant, D., and Chopineau, J. Bordetella pertussis adenylate cyclase toxin translocation across a tethered lipid bilayer. Proc. Natl. Acad. Sci. U.S.A. 2013;110, 20473-20478.

The adenylate cyclase was modified by inactivating the enzymatic activity and rendering it permissive to harbor a variety of polypeptide vaccinal sequences or antigens. This was achieved without altering its high affinity to APC and its capability to translocate into the cytosol of the APC (Fayolle et al., In vivo induction of CTL responses by recombinant adenylate cyclase of Bordetella pertussis carrying viral CD8+ T cell epitopes. J Immunol. 1996;156(12):4697-706).

CyaA recombinant vectors derived from Bordetella *sp.* species CyaA, in particular of *Bordetella pertussis,* have been described as a recombinant vectors able to deliver polypeptides, such as antigens, into the cytosol of APC and were constructed by recombinant DNA technology, especially as described in WO 93/21324 or WO 02/22169. These CyaA vectors are genetically modified adenylcyclase toxoids carrying modifications, such as point mutations, deletions or insertions, which can be obtained using usual site-directed or random mutagenesis techniques.

Originally, CyaA recombinant vectors are constructed by inserting deliver polypeptides into the AC domain at selected residues such as 224-225 or 228-229 or 235-236 or 317-318 as described in EP0637335, but also at 107-108 or 132-133 or 137-138 or 232-233 or 235-236 or 317-318 or 319-320 or 335-336 or 336-337 as described in EP1725259.

A new generation of CyaA recombinant vectors are constructed by truncating the AC domain such as between residues 1 to 373 or residues 1 to 372 as described in EP1317282B1, residues 1 to 300 as described in EP1489092 A1, residues 184 to 320 as EP2875130 A1, residues 228 to 320 as described in EP2690172 A1 or residues 224 to 235 as described in EP2351580 A1.

The CyaA Hly domains were demonstrated to bind specifically to the α_{M}β₂ integrin (CD11b/CD18 (WO02/22169) through its CD11b subunit (Osicka R et al., 2015, eLife, 4:e10766) and so exhibit the capacity to target T-cell epitopes of choice into the CD11b(+) subpopulation of dendritic cells. Immunization of mice with a recombinant CyaA bearing appropriate epitopes led to the induction of strong CD8(+) CTL responses, conferring full protection against a lethal viral challenge and an efficient prophylactic and therapeutic antitumor immunity. In WO02/22169, it has been described that fragments of CyaA encompassing residues 373 to 1706 contain the structure essentially required for interaction with the CD11b/CD18 receptor. More specifically, the amino acid sequence extending from residues 1166 to 1281 comprise a determinant for interaction with the CD11b/CD18 receptor, and more particularly that amino sequence extending from residues 1208 to 1243 are described as critical for the interaction of the toxin with CD11b/CD18 in EP1489092 A1 and EP 1633776 A1.

Some CyaA recombinant vectors are then constructed by combining deletion of AC domain residues such as residues 1 to 300 combined with minor modification of the Hly domain such as acetylation of the Lysine 860 and Lysine 963 residues as illustrated in EP1633776A1 or substitution of the Glutamic acid 570 residue by a glutamine residue and the Lysine 860 by arginine residue as described in EP2233569B1.

Currently the only CyaA recombinant vector used under clinical development is GTL001 (known as ProCervix® in Europe) developed by Genticel as a first-in-class bivalent Human papillomavirus (HPV) therapeutic vaccine candidate in women infected with HPV 16 and/or HPV 18, before the appearance of papillomavirus related cervical lesions or cancer. This vaccine consists of two CyaA proteins carrying E7 antigens of HPV16 and HPV 18 inserted in the CyaA AC domain.

Jiri Masin et al., (Negatively charges residues of the segment linking the enzyme and cytolysin moieties restrict the membrane-permeabilizing capacity of adenylate cyclase toxin, Nature, Scientific Reports 6, Article number:29137) describes that a mutation on a specific part of the Hly domain (between residues 400 to 500 of the CyaA) and especially a substitution of amino acid 445, 446 and 448, have positive impact on the pore forming capacity of the CyaA without altering its AC translocation capacity across target cell plasma membrane. Therefore, some CyaA recombinant vectors can be constructed by combining deletion of AC domain residues with mutation on the Hly domain and especially by amino acid substitution of the residues 445 by an Asparagine (D445N), the residue 446 by an Asparagine (D446N) and of the residue 448 by a Glutamine (E448Q) .

Hence, as used herein, "Adenylate cyclase toxin (CyaA) vector" excludes the native adenylate cyclase toxin. Hence, "Adenylate cyclase toxin (CyaA) vector" means genetically modified adenylate cyclase toxin (CyaA) of *Bordetella sp,* by at least one mutation and/or at least one deletion and/or at least one insertion in the native sequence of *Bordetella sp* (SEQ ID No: 1, 2 or 3). These genetically modifications are
- In one embodiment, located in the AC domain of CyaA, Preferably genetically modifications are deletion in the AC domain of at least 100 amino acids, 200 amino acids, 300 amino acids, more preferably 360 amino acids to 370 amino acids and even more preferably 373 amino acids in the AC domain.
- In another embodiment, located in the Hly domain of CyaA. Preferably genetically modifications are substitutions and especially substitution of amino acid 445, 446 and 448, and even more preferably substitution of the residues 445 by an Asparagine (D445N), the residue 446 by an Asparagine (D446N) and of the residue 448 by a Glutamine (E448Q)

This Adenylate cyclase toxin (CyaA) vector have an amino acid sequence percentage of identity of at least 75% with the amino acid sequence of a native CyaA protein (i.e. CyaA such as SEQ ID NO:1, SEQ ID NO:2 or SEQ ID NO:3) or a fragment thereof, preferably of at least 79%, at least 80%, or at least 85%, still preferably of at least 90%, at least 95%, at least 96%, at least 97%, at least 98%, or at least 99% of identity.

This Adenylate cyclase toxin (CyaA) vector still possesses high affinity to target Antigen Presenting Cells and capability to translocate into the cytosol of the APC and capacity for the delivery of molecules of interest, in particular *Bordetella bronchiseptica* antigen.

In an embodiment the adenylate cyclase toxin (CyaA) vector is a genetically modified adenylate cyclase toxin (CyaA) of *Bordetella pertussis.*

In an embodiment, the adenylate cyclase toxin (CyaA) vector is a genetically modified adenylate cyclase toxin (CyaA) *of Bordetella bronchiseptica.*

In one preferred embodiment, Adenylate cyclase toxin (CyaA) vector comprises or consist of the amino acid sequence of the adenylate cyclase (CyaA) of *Bordetella bronchiseptica or Bordetella pertussis* wherein a *Bordetella bronchiseptica* antigen is inserted in the N terminal part and the AC domain is deleted of at least 100 amino acids, 200 amino acids, 300 amino acids, more preferably 360 amino acids, more preferably 370 amino acids and even more preferably 373 amino acids.

The immunogenic composition comprising a *Bordetella sp.* Adenylate cyclase toxin (CyaA) vector carries at least one antigen of *Bordetella bronchiseptica.*

The term "antigen" generally refers to a protein, a peptide or a polypeptide, which contains at least one epitope to which a cognate antibody can selectively bind; or that can stimulate the production of antibodies or T-cell responses, or both, in an non-human animal, including compositions that are injected or absorbed into this non-human animal. The immune response may be generated to the whole molecule, or to one or more various portions of the protein, the peptide or the polypeptide (e.g., an epitope or hapten). The term "antigen" includes all related antigenic epitopes. An antigen is recognized by antibodies, T-cell receptors or other elements of specific humoral and/or cellular immunity. The term "antigen" includes all related antigenic epitopes. Furthermore, for purpose of the prevention invention, the antigen can be derived, obtained, or isolated from bacteria, more particularly from *Bordetella bronchiseptica* The antigen can consist in a full-length antigen of *Bordetella bronchiseptica* or an antigenic fragment(s).

Typically, the antigen of *Bordetella bronchiseptica* is selected from Bsp22, pertactin, filamentous hemagglutinin (FHA), BteA (also named BopC), or any antigen of *Bordetella bronchiseptica* involved in bacterial colonization or in virulence.

Typically, Bsp22 a self-associating tip complex protein from the type III secretion system (T3SS) of *Bordetella bronchiseptica.*

Typically, pertactin is an autotransporter protein of *Bordetella bronchiseptica* (Inatsuka et al., Pertactin is required for Bordetella species to resist neutrophil-mediated clearance, infection and immunity, July 2010, p. 2901-2909).

Typically, the filamentous hemagglutin (FHA) is an adhesin of *Bordetella bronchiseptica* (Romero et al., Filamentous hemagglutinin of Bordetella pertussis: a key adhesion with immunomodulatory properties? Future Microbiol. (2014) 9(12), 1339-1360).

Typically, BteA also named BopC is a protein secreted from the *Bordetella bronchiseptica* type III secretion system (Kuwae et al., BteA Secreted from the Bordetella bronchiseptica Type III Secretion System Induces necrosis through an Actin Cytoskeleton Signaling Pathway and Inhibits Phagocytosis by Macrophages, PLOS ONEDOI:10.1371/journal.pone.0148387, February 2016), (Kuwae et al., BopC is a novel type III effector secreted by Bordetella bronchiseptica and has a critical role in type III-dependent necrotic cell death, the journal of biological chemistry vol. 281, NO. 10, pp. 6589-6600, March 10, 2006).

Preferably, the antigen of *Bordetella bronchiseptica* is the Bsp22 antigen.

As used herein, the term "Bsp22 antigen" includes whole Bsp22 antigen and any Bsp22 antigenic fragments.

In one embodiment, the amino acid sequence of Bsp22 antigen is described in SEQ ID No: 4.

In another embodiment, Bsp22 antigen is conjugated to a linker.

In another preferred embodiment, the amino acid sequence of Bsp22 antigen conjugated to the linker is described in SEQ ID No: 5.

In another embodiment, Bsp22 antigen is any polypeptide having an amino acid identity between 60% and 100%, preferably more than 80% and even preferably more than 90%, with a part of SEQ ID NO:5 having the same size.

In a most preferred embodiment, the sequence of the *Bordetella bronchiseptica* adenylate cyclase toxin (CyaA) vector carrying the Bsp22 antigen from *Bordetella bronchiseptica* is as set forth in SEQ ID NO: 6.

In yet another embodiment, the immunogenic composition further comprises at least one adjuvant.

The term "adjuvant" as used herein refers to a compound or mixture that enhances the immune response when administered together with an immunogen or antigen.

Typically the adjuvant is chosen among Complete Freund's Adjuvant (CFA), Incomplete Freund's Adjuvant (IFA), montanide ISA (incomplete seppic adjuvant), muramyl peptides such as muramyl dipeptide (MDP) MDP-Lys (L18) (N.sup..alpha.-acetylemuramyl-L-alanyl-D-isoglutaminyl-N.sup.esteoroyl-L--lysine), zinc sulphate, colloidal iron hydroxide, calcium phosphate or calcium chloride, CpG oligodeoxynucleotides (CPG ODN) such as CPG ODN 1826 and CPG ODN 2007, MF59 which is a detergent stabilized oil-in water emulsion containing 5% squalene (w/v), 0.5% Tween.RTM. 80 (w/v) and 0.5% Span (w/v) in water, polysaccharides (such as Inulin) and liposomes (such as cationic liposomes, ISCOMs), molecules which have the capacity to activate T-cell immune response, PRP (Pathogen Recognition Receptors), such as adjuvants that bind or are agonists to TLR (Toll like receptor) 3, 4, 7, 8 and/or 9 on immune cells (such as APC).

In a particular embodiment, the adjuvant is a TLR ligand, in particular a TLR ligand selected from the group consisting of TLR ligands of class 3, such as Poly IC, Poly-ICLC, TLR ligands of class 4, such as MPL or GLA, TLR ligands of class 9, such as CpG, and TLR ligands of class 7/8, such as Imiquimod.

In a most preferred embodiment, adjuvant is Poly-ICLC. Poly-ICLC can be purchased from Oncovir Inc, (WA, US) as HiltonolTM.

Moreover, the immunogenic composition as defined herein may be combined or mixed with at least one immunopotentiator, such as at least another adjuvant, or a surfactant or immunomodulatory substances, such as cytokines or chemokines, or a growth factors such as such as GM-CSF or a combination thereof.

By "combined", it is meant that the immunogenic composition as defined herein and the immunopotentiator are both put in contact with the host, at the same or different time and/or by the same or different modes of administration, preferably at the same site of contact.

In contrast, "mixed" means that the immunogenic composition as defined herein and the immunopotentiator are in the same formulation when administered.

The amounts and concentrations of adjuvants useful in the context of the present invention can readily be determined by the skilled artisan.

In a particularly preferred embodiment, the immunogenic composition comprises a *Bordetella bronchiseptica* adenylate cyclase toxin (CyaA) vector carrying a Bsp22 antigen, a Poly-ICLC as adjuvant and optionally some pharmaceutically/veterinary-acceptable carriers.

Immunogenic composition and vaccines encompassed by the present invention can further include one or more pharmaceutically/veterinary-acceptable carriers.

A used herein, "pharmaceutically/veterinary-acceptable" refers to those compounds, materials, excipients, compositions or dosage forms which are, within the scope of sound medical judgment suitable for use in contact with the tissues of subjects, especially veterinary subjects, without excessive toxicity, irritation, allergic response or other problem complications commensurate with a reasonable benefit/risk ratio. Pharmaceutically/veterinary acceptable carriers include but are not limited to solvents, dispersion media, coatings, stabilizing agents, diluents, preservatives, antibacterial and antifungal agents, isotonic agents, adsorption delaying agents, and the like. Diluents can include water, saline, dextrose, ethanol, glycerol and the like. Isotonic agents can include sodium chloride, dextrose, mannitol, sorbitol and lactose, among others known to those skilled in the art. Stabilizers include albumin. Preservatives include merthiolate.

In a preferred embodiment, the immunogenic composition is for use for treating a *Bordetella bronchiseptica* infection in a non-human animal

The term "treatment" or "method of treating" or its equivalent is not intended as an absolute term and, when applied to, for example, infection refers to a procedure or course of action that is designed to reduce or eliminate or to alleviate the symptoms of an infection. A "treatment" or a "method of treating" infection does not necessarily mean that the infection will, in fact, be eliminated, be reduced, or that the symptoms of infection will, in fact be alleviated. Often, a "treatment" or a "method of treating" infection will be performed even with a low likelihood of success but is nevertheless deemed to induce an overall beneficial course of action. In an embodiment, the term "treatment" also includes the prevention of an infection. Instead, prevention, e.g., of *Bordetella bronchiseptica* infection, refers to delay on onset, reduced frequency of symptoms, or reduced severity of symptoms associated with the disorder. Prevention therefore refers to a broad range of prophylactic measures that will be understood by those in the art. In some circumstances, the frequency and severity of symptoms is reduced to non-pathological levels. In another embodiment, the term "treatment" also includes a prophylactic treatment of non-human animals. A prophylactic treatment aims at preventing the *Bordetella bronchiseptica* infection of said non-human animal and/or preventing the apparition, the development and/or the occurrence of a pathological state and /or symptoms in said non-human animal. The prophylactic treatment encompasses vaccination.

Typically, the term "non-human animal" encompasses, and preferably is, a non-human mammal such as for example a canine, a feline, a bovine, an ovine, a porcine, a camelid or an equine.

Preferably, the non-human animal is a canine or a feline.

In a more preferred embodiment, the immunogenic composition is for use for treating canine tracheobronchitis, commonly named kennel cough.

Typically, the immunogenic composition is administered in a non-human animal by different routes: by oral or per oral administration, oronasal administration, parenteral administration, intranasal administration, respiratory administration.

"Oral" or "peroral" administration, as used herein, refers to the introduction of the immunogenic composition of the invention, into the non-human animal body through or by way of the mouth and involves swallowing or transport through the oral mucosa (e.g., sublingual or buccal absorption) or both. Intratracheal is also a means of oral or peroral administration.

"Oronasal" administration, as used herein, refers to the introduction of the immunogenic composition of the invention, into the non-human animal body through or by way of the nose and the mouth, as would occur, for example, by placing one or more droplets in the nose. Oronasal administration involves transport processes associated with oral and intranasal administration.

"Parenteral administration", as used herein, refers to the introduction of the immunogenic composition of the invention, into the non-human animal body through or by way of a route that does not include the digestive tract. Parenteral administration includes subcutaneous, intramuscular, intraarterial, and intravenous administration. For the purposes of this disclosure, parenteral administration excludes administration routes that primarily involve transport of the substance through mucosal tissue in the mouth, nose, trachea, and lungs.

"Intranasal" administration, as used herein, refers to the introduction of the immunogenic composition of the invention, into the non-human animal body through or by way of the nose, and involves transport of the substance primarily through the nasal mucosa.

"Respiratory" administration, as used herein, refers to the introduction of the immunogenic composition of the invention, into the non-human animal body through or by way of inhalation of a nebulized (atomized) substance. In respiratory administration, the primary transport mechanism involves absorption of the atomized substance through the mucosa in the trachea, bronchi, and lungs and is therefore different than intranasal or peroral administration.

The immunogenic compositions defined herein may be in a solid form (capsule, powder, tablet, pill, suppository, quick release tablet, gastro-resistant tablet, delayed release tablet), a powder form, preferably after lyophilization (lyophilized form or lyophilized powder form) which needs to be reconstituted for example with diluents(s) before injection, or in a liquid form, such as an injectable solution or injectable suspension.

Preferably, the immunogenic composition of the invention is administered to a non-human animal subcutaneously in a liquid form.

In another embodiment, the immunogenic composition of the invention is administered to a non-human animal with another vaccine, selected from vaccines for treating others pathogens in *Bordetella bronchiseptica* infection such as p*asteurella multocida,* herpesvirus, calicivirus, mycoplasma, chlamydia, adenovirus and parainfluenza virus alone or in combination.

In another embodiment, the immunogenic composition of the invention can be administered to the non-human animal in one dose or in multiple doses.

"Dose" refers to the immunogenic composition given to a subject. A "first dose" or "priming dose" refers to the dose of such composition given on day 0. A "second dose" or a "third dose" or an "annual dose" refers to an amount of such composition given subsequent to the first dose, which can be but is not required to be the same vaccine or immunogenic composition as the first dose.

A particular regimen that may be adopted is a repeated administration protocol, especially in a protocol which encompasses two rounds or more of administration.

Typically, the immunogenic composition is administered in one dose, followed by subsequent doses.

Preferably, the immunogenic composition is administered in one dose, followed by a second dose.

More preferably, the immunogenic composition is administered in first dose, followed by a second dose, the second dose is given 3 weeks after the first dose.

In another preferred embodiment, the first dose and the second dose are administered subcutaneously.

The present invention also relates to an immunogenic composition comprising a *Bordetella sp.* adenylate cyclase toxin (CyaA) for use for treating a *Bordetella bronchiseptica* caused infection and / or related disease.

In one preferred embodiment, the *Bordetella bronchiseptica* caused infection and / or related disease is tracheobronchitis, commonly named kennel cough.

Preferably, the adenylate cyclase toxin (CyaA) is from *Bordetella bronchiseptica.*

The invention also concerns a method for treating of a non-human animal presenting with a *Bordetella bronchiseptica* infection or suspected to have a *Bordetella bronchiseptica* infection comprising (a) the administration of an effective amount of the immunogenic composition of the present invention into said non-human animal, possibly as multiple administered doses, and (b) the follow up of the condition of said non-human animal.

The invention also concerns a method for treating of a non-human animal presenting with a *Bordetella bronchiseptica* infection or suspected to have a *Bordetella bronchiseptica* infection comprising (a) the administration of an effective amount of the immunogenic composition of the present invention into said non-human animal, possibly as multiple administered doses, and (b) the follow up of the condition of said non-human animal, possibly as multiple administered doses.

An "effective amount" of the immunogenic composition of the invention refers to the effective amount sufficient to prevent, reduce, eliminate, control, treat or inhibit the *Bordetella bronchiseptica* infection and/or the symptoms. The term "controlling" is intended to refer to all processes wherein there may be a slowing, interrupting, arresting, or stopping the progression of the diseases and conditions described herein, but does not necessarily indicate a total elimination of all disease and condition symptoms. The doses used for the administration can be adapted as a function of various parameters, in particular as a function of the mode of administration used, of the relevant pathology, or alternatively of the desired duration of treatment. Naturally, the form of the pharmaceutical composition, the route of administration, the dosage and the regimen naturally depend on the condition to be treated, the severity of the illness, the age, weight, and sex of the subject, etc. The ranges of effective doses provided below are not intended to limit the invention and represent preferred dose ranges. However, the preferred dose can be tailored to the individual subject, as is understood and determinable by one of skill in the art, without undue experimentation.

For example, the amount of active agent present in the immunogenic composition of tbe present invention will vary depending upon the particular active agent used, as well as the intended subject for administration of the composition, and route of administration. In general, the immunogenic composition of the present invention will comprise from about 0.01% to about 90% by weight active agent, preferably from about 10% to about 60% and more preferably, from about 30% to about 50%, based upon the total weight of the immunogenic composition taken as 100% by weight.

In another aspect, the invention concerns a method for treating an infection in a non-human animal preferably in a canine and/or a feline, wherein the method comprises administering an effective amount of the immunogenic composition of the present invention to a non-human animal, preferably a canine or a feline, in need of such treatment. All the embodiments disclosed above for the immunogenic composition are encompassed in this aspect.

In yet another embodiment, the present invention relates to a method for treating a *Bordetella bronchiseptica* infection, preferably, a method for treating canine tracheobronchitis infection commonly called kennel cough, wherein the method comprises administering a therapeutically effective amount of the immunogenic composition of the invention to a canine in need of such treatment. All the embodiments disclosed above for the immunogenic composition are encompassed in this aspect.

In another aspect, the present invention relates to the use of *Bordetella sp.* preferably *Bordetella bronchiseptica* adenylate cyclase toxin (CyaA) vector for the treatment of *Bordetella bronchiseptica* infections and more preferably the canine tracheobronchitis infection commonly called kennel cough.

In a further aspect, the present invention concerns the use of an immunogenic composition comprising a *Bordetella sp.* adenylate cyclase toxin (CyaA) vector with at least one *Bordetella bronchiseptica* antigen for the treatment of *Bordetella bronchiseptica* infection in a non-human animal. In yet another embodiment, the present invention relates to the use of an immunogenic composition for the treatment of a *Bordetella bronchiseptica* infection comprising a *Bordetella sp.* adenylate cyclase toxin (CyaA) vector with a *Bordetella bronchiseptica antigen* for the treatment of the canine tracheobronchitis infection commonly called kennel cough.
All the embodiments disclosed above for the immunogenic composition are encompassed in this aspect.

In another aspect, the present invention related to the use of *Bordetella sp.* preferably *Bordetella bronchiseptica* adenylate cyclase toxin (CyaA) vector for the manufacture of a pharmaceutical preparation for the treatment of the canine tracheobronchitis infection commonly called kennel cough.

In a last aspect, the present invention concerns the use of an immunogenic composition comprising a *Bordetella sp.* adenylate cyclase toxin (CyaA) vector with at least one *Bordetella bronchiseptica* antigen for the manufacture of a pharmaceutical and/or vaccinal preparation for the treatment of an *Bordetella bronchiseptica* infection in a non-human animal All the embodiments disclosed above for the immunogenic composition are encompassed in this aspect.

In a further embodiment, the invention relates to the use of an immunogenic composition for the manufacture of a pharmaceutical preparation for the treatment of a *Bordetella bronchiseptica* infection, comprising a *Bordetella sp.* adenylate cyclase toxin (CyaA) vector with at least one *Bordetella bronchiseptica* antigen for the treatment of the canine tracheobronchitis infection commonly called kennel cough. All the embodiments disclosed above for the immunogenic composition are encompassed in this aspect.

### EXAMPLES

### Example 1: Construction, production and purification of CyaA toxoid.

Antigens: The synthetic peptide corresponding to antigens such as Bsp22 of Bordetella Bronchiseptica (SEQ ID No: 4) was purchased from Polypeptide (Strasbourg, France).

Construction of recombinant CyaA toxoid: Recombinant CyaA toxoid comprising the antigens such as Bsp22 (CyaA-Bsp22) genetically link to the N-terminal part of Bordetella Bronchiseptica CyaA protein (SEQ ID NO:6) were produced in E. coli XL1-Blue (Stratagen, an Agilent Technologies Company, USA) transformed with appropriate pT7CACT1-derived constructs (Osicka R. et al., 2000, Infect. Immun., 68: 247-256). Exponential 500-ml cultures were grown at 37°C and induced by isopropyl 1-thio-β-D-galactopyranoside (IPTG, 1 mM) for 4 h before the cells were washed with 50 mM Tris-HCl (pH 8.0), 150 mM NaCl, resuspended in 50 mM Tris-HCl (pH 8.0), 0.2 mM CaCl2, and disrupted by sonication. Upon centrifugation at 25,000 x g for 20 min, the insoluble cell pellets were resuspended in 8 M urea, 50 mM Tris-HCl (pH 8.0), 50 mM NaCl, 0.2 mM CaCl2. Upon centrifugation at 25,000 x g for 20 min, clarified urea extracts were loaded onto a DEAE-Sepharose column equilibrated with 8 M urea, 50 mM Tris-HCl (pH 8.0), 120 mM NaCl. After washing, the CyaA toxoid were eluted with 8 M urea, 50 mM Tris-HCl (pH 8.0), 2 M NaCl, diluted four times with 50 mM Tris-HCl (pH 8.0), 1 M NaCl buffer, and further purified on a phenyl-Sepharose column equilibrated with the same buffer. Unbound proteins were washed out with 50 mM Tris-HCl (pH 8.0), and the CyaA toxoid were eluted with 8 M urea, 50 mM Tris-HCl (pH 8.0), 2 mM EDTA and stored at - 20°C. Concentrations of the purified CyaA toxoid were determined by the Bradford assay (Bio-Rad, Hercules, USA) using bovine serum albumin as a standard. All purified CyaA - Bsp22 toxoid were more than 90% pure as judged by SDS-gel analysis.

### Example 2: Materials and Methods for the evaluation of the immunogenic composition

This study was approved by the Ethical Committee of MENSER (number 201509155295755).

### Animals

Beagle dogs were purchased from a licensed kennel for animal breeding and husbandry (CEDS, Centre d'Elevage du Domaine de Souches, France). Specific pathogen-free puppies were eight week old at their arrival. They were bred in a specific facility free of Bordetella bronchispetica. Before the beginning of the study, all the puppies were shown to be seronegative for *Bordetella bronchiseptica.* All dogs were dewormed and vaccinated against canine distemper virus, parvovirus, leptospirosis, infectious canine hepatitis and parainfluenza virus. They were housed in an accredited class II facility and were provided commercial dog food (VetCare Nutrition, Junior 10kg, Royal Canin) and tap water ad libitum.
In total, 15 dogs were used, 8 females and 7 males. Dogs were randomly allocated to two groups, following an equivalent ratio of male vs female in each group. The control group was composed of 5 puppies and the vaccinated group 10

### Vaccination preparation, formulation and regimens

Dogs were vaccinated with the CyaA-Bsp22 toxoid as prepared in Example 1.

Each vaccinated dog received 2 subcutaneous injections of 300µg CyaA-Bsp22 toxoid (in Urea 2.4M) and 150µg of Poly-ICLC (Oncovir) at 3 weeks interval ("vaccine group"). Control dogs received the same amount of adjuvant (Poly-ICLC) in 2.4M Urea ("control group").

All dogs received two parenteral vaccine injections at 10 weeks of age (2 weeks after their arrival) and three weeks later (D0 and D21). The control group received the same amount of excipient and adjuvant as in the vaccine formulation.

All dogs were infected by *Bordetella bronchiseptica* four weeks after the second vaccine injection (D49). All puppies were exposed to an aerosol of 1 ml of challenge preparation (8 109 CFU/ml) during 15 min using a nebuliser (OMRON, compAir Pro C900). Puppies were slightly tranquilized with an IM injection of a mix of butorphenol (0.3mg/kg) and acepromazine (0.01mg/kg) 10 min before the aerosol nebulization.

### Bacterial strains and infection procedure

*Bordetella bronchiseptica* strains were purchased from the Pasteur Institute Collection (CRBIP4.201 and CRBIP4.202) and ATTC (Bb 31124 Moreno-Lopez). Strains were inoculated on Bordet Gengou agar plates supplemented with 15% defibrinated sheep blood (Biomerieux, France) and incubated aerobically for 48 hours at 37°C. Strains were then transferred to Stainer-Scholte broth and incubated, with shaking, under aerobic conditions for 18 hours at 37°C. The cultures were stopped when the DO at 600nm reached 1. The bacterial count at D.O.=1 was determined to be 109 cfu/ml.

The challenge preparation was an equal mix of the three bacterial strains, corresponding to a total of 8.0 109CFU/ml.

### Clinical follow-up

Two clinical follow-up were performed.

A clinical follow-up was performed daily from the day before each vaccine injection and until 7 days post vaccination following the criteria presented in table 1.

**TABLE 1**

| | | | | | |
|---|---|---|---|---|---|
| Rectal Temperature (°C) : | | | | | |
| Weight (kg) : | | | | | |
| Lethargy | | | | | |

| | MACROSCOPIC ANALYSES | | | | |
|---|---|---|---|---|---|
| | ***Erythema*** | ***Induration*** | ***Oedema*** | ***Erosionl Ulceration*** | ***Other problems*** |
| CLINICAL SIGNS | Absent | Absent | Absent | Absent | |
| | Mild | Mild | Mild | Mild | |
| | Severe | Severe | Severe | Severe | |

A clinical follow-up was performed daily from the day before challenge and during one month post infection following the criteria presented in table 2.

**TABLE 2**

| | | |
|---|---|---|
| Rectal Temperature (°C) : | | |
| Weight (kg) : | | |

| | **Qualifier** | **Score** |
|---|---|---|
| **Lethargy** | | |
| **Cough** | **Absente** | **0** |
| | **Induced** | **1** |
| | **Spontaneous** | **3** |
| | | |
| **Nasal Discharge** | **Clear** | **1** |
| | **Thick** | **2** |
| **Sneezing** | **Frequent** | **1** |
| **Vomiting** | | **5** |
| **Dyspnea** | | **10** |

### Isolation of PBMC (Peripheral Blood Mononuclear Cells)

Sampling was performed every week, starting on the day of the first immunization until day 70 after the primo-vaccination. Blood samplings were performed at the jugular vein and blood was collected in heparinized tubes. Seven dogs (3 controls and 4 vaccinated) were chosen at random at their arrival for the follow-up of the cellular immune response.

PBMC were immediately isolated from whole blood by density gradient centrifugation using Histopaque 1077 (density: 1.077 g/mL, Sigma-Aldrich).

After centrifugation and washings, the resulting cell pellet was suspended in a small volume of complete medium and the viable cell concentration was determined by microscopic examination using Gibco® trypan blue exclusion. Viability was >90% in all samples.

### IFNγ ELISPOT

The cells were cultured in complete medium composed of Gibco® Roswell Park Memorial Institute (cRPMI) Medium 1640 containing GlutaMAX® (L-alanyl-L-glutamine) and HEPES, supplemented with 1% sodium pyruvate, 1% non-essentials aminoacids, 100 µg/ml streptomycin, 100 IU penicillin and 10% heat-inactivated Gibco® Fetal Bovin Serum (cat 10270, batch 41G7832K) at a concentration of 2 x 10⁶ PBMC/ml. PBMC were incubated in triplicate in 96 well PVDF plates (Mabtech) previously coated with 0.75 µg/well of equine IFN 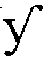 capture antibody (bIFN 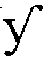 -I) at 37 °C, 5% CO2 for 20-24h.

For each dog and at each time point, cells were cultured in complete medium as a negative control and in the presence of 50 ng/ml Phorbol 12-Myristate 13-Acetate and 1 µM ionomycine calcium salt (Sigma-Aldrich) as a non-antigen specific positive controls to assess the functionality of the PBMC. The antigen-specific cellular response against the vaccine was tested with 0.5 µg/well of CyaA or 0.5 µg/well of Bsp22.

Plates were washed five times with PBS and 0.025 µg specific anti equine IFNγ biotinylated antibody (Mabtech) was incubated 2 hours at room temperature. After five washed, plates were incubated with 1:1000 streptavidin-ALP (Mabtech) and BCIP/NBT chromogen (Mabtech). The number of spots was determined with an automated ELISpot reader (CTL®).
For each point, the geometric mean of spot number was calculated (mean of triplicate). For the specific response against CyaA or Bsp 22, the data are presented as the number of spots per 10⁶ cells after stimulation with CyaA (or Bsp22) minus the value obtained with the negative control obtained with medium alone the same day.

### ELISA (Enzyme-Linked ImmunoSorbent Assay)

Blood samplings were performed at the jugular vein and blood was collected in uncoated tubes in all dogs. Sera were stored at -80°C until tested.

ELISA testing was performed every week, starting on the day of the first vaccination until day 70 post-vaccination to measure the level of total IgG, IgG1 and IgG2 antibodies specific for CyaA and Bsp 22.

Briefly, the technique was performed as follow.
A 96 well MAXISORP plate (Nunc) was coated with 0.25 µg of purified CyaA or 0.5 µg Bsp 22 per well in carbonate buffer (pH 9.6) overnight at +4°C. Nonspecific sites were blocked with PBT/BSA (PBS + 0.1% Tween-20 + 1% Bovine Serum Albumin) for 2 hours at room temperature. Serial two-fold dilutions of sera were tested starting at 1/5000 until 1/40000. The first dilution that did not saturate the ELISA in vaccinated dogs was used to analyse the results. After 1 hour of incubation at room temperature, plates were washed five times with PBT. A specific peroxidase-conjugated rabbit polyclonal anti-dog IgG secondary Ab (304-035-0030, Jackson Immune Research) was added at 0.04 µg/well and incubated 1 hour of incubation at room temperature. After five washed, revelation was done with incubation with TMB Liquid Substrate System (Sigma).

For ELISA detecting dog IgG1 or IgG2, the protocol was similar. A specific peroxidase-conjugated goat polyclonal anti-dog IgG1 secondary Ab (Bethyl Laboratory) was added at 0.02µg/well and a specific peroxidase-conjugated sheep polyclonal anti-dog IgG2 secondary Ab (Bethyl Lab) was added at 0.002 µg/well.

Plates were read at 450 nm with µquant (Biotek). To standardize the results between plates, a positive dilution range was used on each plate.

### Statistical analysis

All statistical analyses were performed using the following software: www.socscistatistics.com/tests/mannwhitney/Default2.aspx. The significant threshold was set at p=0.05. Intergroup comparisons of the results were performed using a Mann Witney test.

### Example 3: Safety of the immunogeniccomposition comprising CyaA vector carrying Bsp22 antigen

In order to evaluate the tolerance of the vaccine, complete general and local clinical examinations were performed the day before and after each injection once a day for 7 days. Examinations were performed according to Table 1.

Immunized dogs did not present alterations of their general condition. No sign of lethargy or appetite loss was identified. No sign of hyperthermia was detected, rectal temperature ranges between 37.6 and 39.3 for all puppies.

At the injection site, no local reaction was observed, neither after the first injection nor after the second injection.

These results demonstrated that the candidate vaccine was very well tolerated in young puppies.

The immunogenic composition comprising CyaA vector carrying Bsp22 antigen was confirmed to be safe.

### Example 4: Efficacy of the immunogeniccomposition comprising CyaA vector carrying Bsp22 antigen

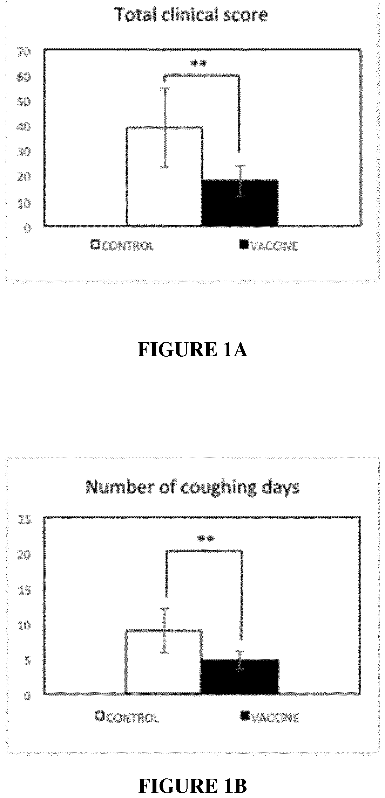

Clinical follow-up of puppies after *Bordetella bronchiseptica* challenge was performed according to the criteria listed in Table 2 (FIGURE 1).

The infection did not induce hyperthermia in both groups of dogs, and no differences in body temperature were observed between vaccinated and control puppies (data not shown).

After infection, control puppies display clear clinical signs of tracheobronchitis, including spontaneous and induced coughing, nasal discharge and, for some of them, vomiting. All dogs spontaneously recovered from infection in three weeks.

The mean (± SD) of cumulative score was determined for each group of puppies. P<0.01 between vaccinated (n=10) and control group (n=5) following table 2 criteria (FIGURE 1A). Total clinical score was significantly reduced (p<0.01) in the vaccine group.

For each dog, the number of coughing days was noted (FIGURE 1B). Results represent the mean (±SD) for each group of puppies. P<0.01 between vaccinated (n=10) and control group (n=5).

The number of coughing days was significantly reduced (p<0.01) in the vaccine group.

Hence, the immunogenic composition comprising CyaA vector carrying Bsp22 antigen delays the outbreak of tracheobronchitis and reduces the duration and intensity of cough.

The immunogenic composition reduces the intensity and duration of clinical signs after experimental infection.

### Example 5: immunogenicity of the immunogeniccomposition comprising CyaA vector carrying Bsp22 antigen

### Cellular Th1 response

The IFNγ production of PBMC after specific *in vitro* re-stimulation was quantified by ELISpot, in order to monitor the cellular response induced by CyaA.

The plated cells with Phorbol Myristate Acetate plus ionomycine (PMA+Io) were stimulated to induce a polyclonal T-cell activation (positive control) in order to assess the functionality of our assay. Non-specific activation was detected with cells cultured in the presence of the medium only (negative control).

For each time point and each puppy, an average of 4.3 10³spots per 10⁶ cells could be detected after PMA+Io stimulation (data not shown), while no spots were detected when cells were cultured in medium alone. These responses were homogeneous among dogs, independently of their status (vaccinated versus placebo) and at the different time points.

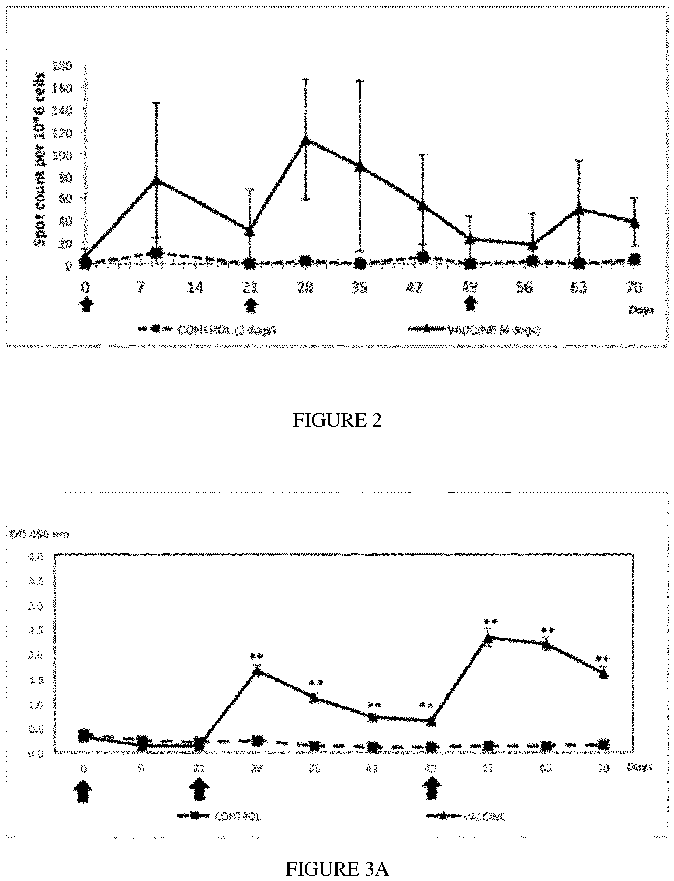

Specific IFNγ production by PBMC after CyaA stimulation was determined by ELISpot in control (n=3) and vaccinated (n=4) dogs once a week after vaccination and infection (FIGURE 2). Results are presented as the mean of spot numbers (±SD) per 10⁶ cells for each group. Arrows indicate the times of vaccination and infection.

After *in vitro* PBMC re-stimulation with CyaA, we observed an early and significant production of IFNγ, as early as 9 days after the first injection between vaccine and control groups (FIGURE 2).

This IFNγ production was rapidly boosted by the second injection, with a peak of IFNγ production one week after the second injection, at day 28. At the time of infection (D49), we were still able to detect a cellular response against CyaA in vaccinated puppies. Interestingly, 2 weeks after the infection, the cellular response against CyaA was significantly boosted in vaccinated dog while non-vaccinated puppies did not mount a specific cellular response. Because of the small number of animals in each group, statistical analyses were not possible.

Altogether, our results demonstrate that CyaA induces a specific cellular response skewed toward a Th-1 phenotype in dogs.

### Humoral response

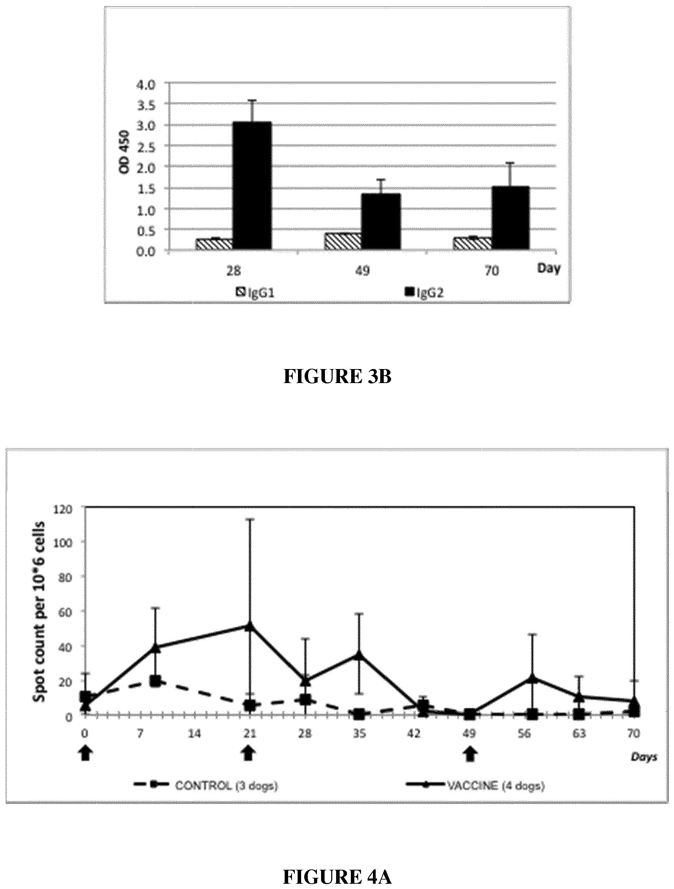

The humoral response induced against CyaA was monitored every week after the first vaccination (FIGURE 3).

Over the course of the study, all vaccinated dogs developed a strong humoral response against the CyaA protein (FIGURE 3A). This response was detectable one week after the second vaccine injection and was significantly higher than in control dogs during the course of the study (p<0.01). Interestingly, this humoral response was boosted by the virulent challenge in vaccinated puppies while the infection did not induce anti-CyaA antibody synthesis in non-vaccinated puppies.

In order to determine the profile of this humoral response, a CyaA ELISA specific for IgG1 and IgG2 was performed. IgG1 and IgG2 specific for CyaA were detected in vaccinated dog at the peak of the humoral response one week after the second vaccine injection (D28), just before the infection (D49) and at the end of the experiment (D70) (FIGURE 3B). The results clearly indicate that the humoral response was almost exclusively orientated toward the IgG2 subclass, confirming that the vaccine orientated the CyaA response toward a Th-1 phenotype.

### Specific immune response against Bsp22 antigen

The next step for the validation of the immunogenicity of the candidate vaccine is to demonstrate that it also induces a specific cellular response against the target antigen included in the vaccine i.e. the Bsp22 protein. The IFNγ production was thus quantified by ELISpot by PBMC after *in vitro* Bsp22 specific re-stimulation.

A rapid cellular response detectable was observed as soon as 9 days after the first vaccination (FIGURE 4A). Interestingly, the second vaccine did not boost this cellular response. On the opposite, one week after the infection, a boost of the specific cellular response was detectable in vaccinated puppies. No response against Bsp22 was observed in control puppies after the infection. Because of the small number of animals in each group, statistical analyses were not possible.

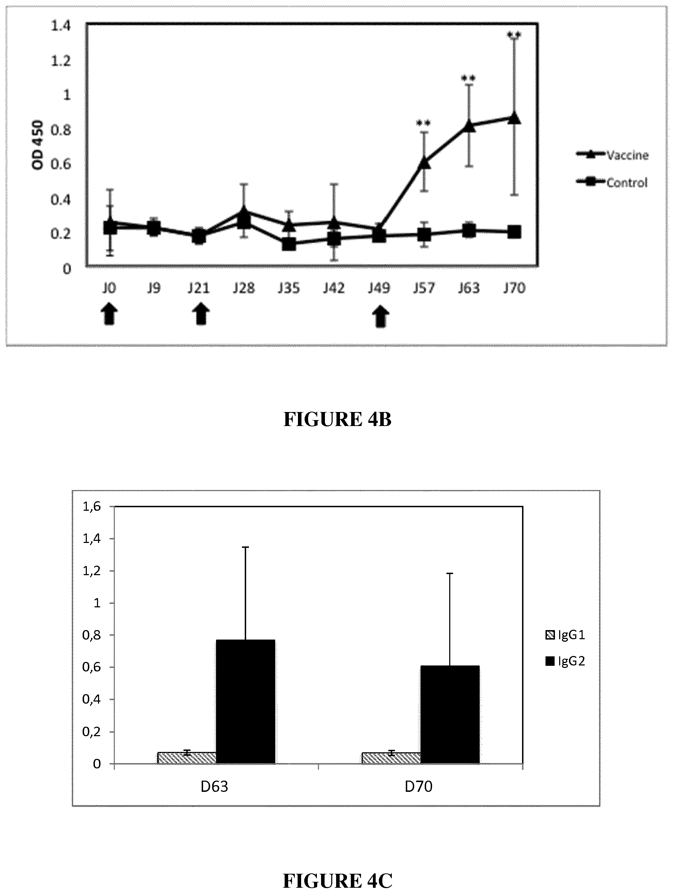

It has been checked whether a humoral response was also induced against Bsp22 in vaccinated animals. Bsp22-specific IgG Ab were not detectable in vaccinated puppies before the infection (FIGURE 4B). However, one week after the infection, we observed a significant and rapid increase in the level of total IgG specific for Bsp22 in the serum of vaccinated puppies (p<0.01). Control dogs did not produce IgG specific for Bsp22 after the infection.

To further qualify this response, the subtype of IgG produced by vaccinated puppies after the infection was determined. IgG1 specific for Bsp22 were not detectable while a strong IgG2 response was observed (FIGURE 4C).

These results demonstrate that CyaA can efficiently present Bsp22 antigen to the immune system of puppies. The response induced is orientated toward a Th-1 phenotype, characterized by IFNγ production and IgG2 synthesis. In addition, this response is boosted by the infection.

## Claims

1. An immunogenic composition comprising a Bordetella sp. adenylate cyclase toxin (CyaA) vector carrying at least one antigen of Bordetella bronchiseptica.

2. The immunogenic composition according to claim 1, wherein the adenylate cyclase toxin (CyaA) vector is selected from a Bordetella bronchiseptica adenylate cyclase toxin (CyaA) vector or a Bordetella pertussis adenylate cyclase toxin (CyaA) vector.

3. The immunogenic composition according to claim 1 or 2 wherein the adenylate cyclase toxin (CyaA) vector is a Bordetella bronchiseptica adenylate cyclase toxin (CyaA) vector.

4. The immunogenic composition according to anyone of the preceding claims, wherein the at least one antigen of Bordetella bronchiseptica is selected from Bsp22, pertactin, filamentous hemagglutinin (FHA), BteA, or any antigen of Bordetella bronchiseptica involved in bacterial colonization or in virulence.

5. The immunogenic composition according to anyone of the preceding claims, wherein the antigen of Bordetella bronchiseptica is a Bsp 22 antigen.

6. The immunogenic composition according to claim 5, wherein Bsp22 antigen is conjugated to a linker.

7. The immunogenic composition according to claim 6, wherein the Bsp22 antigen conjugated to a linker is as set forth in SEQ ID NO: 5.

8. The immunogenic composition according to anyone of claim 5 to 7, wherein the Bordetella bronchiseptica adenylate cyclase toxin (CyaA) vector carrying Bsp22 antigen of Bordetella bronchiseptica is as set forth in SEQ ID NO:6.

9. The immunogenic composition according to anyone of the preceding claims, further comprising at least one adjuvant, preferably the adjuvant is a TLR ligands selected from the group consisting of TLR ligands of class 3, TLR ligands of class 4, TLR ligands of class 9, and TLR ligands of class 7/8, and more preferably the adjuvant is a Poly IC-LC.

10. An immunogenic composition according to anyone of claims 1 to 9 for use for treating Bordetella bronchiseptica infections and related diseases in a non-human animal.

11. The immunogenic composition according to claim 10, wherein the non-human animal is a canine or a feline.

12. The immunogenic composition according to claim 10 or 11, wherein the Bordetella bronchiseptica infection is canine tracheobronchitis.

13. An immunogenic composition comprising a Bordetella sp. Adenylate cyclase toxin (CyaA) for use for treating Bordetella bronchiseptica infections and related diseases in non-human animals.

14. The immunogenic composition according to claim 13, wherein the adenylate cyclase toxin (CyaA) is a Bordetella bronchiseptica adenylate cyclase toxin (CyaA).

15. The immunogenic composition according to claim 13 or 14, wherein the Bordetella bronchiseptica infection is canine tracheobronchitis.
